# EUROPEAN PATENT APPLICATION

(11) **EP 1 354 552 A1**
(43) Date of publication of application: **22.10.2003**
(21) Application number: 01271884.7
(22) Date of filing: 26.12.2001
(51) Int. Cl.: A61B 5/02, A61B 5/11

(54) **HEALTH CONTROL SYSTEM, HEALTH CONTROL DEVICE, SERVER AND HEALTH CONTROL METHOD**

(30) Priority: 26.12.2000 JP 2000404815; 27.12.2000 JP 2000404973
(71) Applicant: ARKRAY, Inc., Kyoto-shi, Kyoto 601-8045 (JP)
(72) Inventor: NISHI, Kenzo, Osaka-shi, Osaka 534-0016 (JP); TAMURA, Katsuhiko, ARKRAY, INC., Kyoto-shi, Kyoto 601-8045 (JP)
(74) Representative: Piésold, Alexander J.
(86) International application number: JP0111441
(87) International publication number: WO02051308

(57) **Abstract**

A healthcare system is composed of a healthcare apparatus (1) for obtaining health-related information of a user and a server (2) for creating exercise menu information to be a target for the user to do exercise and advice information for healthcare of a user. The healthcare apparatus (1) transmits the obtained health-related information to the server (2). The server (2) creates exercise menu information and transmits the created exercise menu information to the healthcare apparatus (1), before the healthcare apparatus (1) obtains health-related information. Furthermore, after receiving the health-related information, the server (2) creates advice information based on the exercise menu information and the received health-related information, and transmits the created advice information to the healthcare apparatus (1).

## Description

### Technical Field

The present invention relates to a healthcare system for obtaining health-related information such as the number of steps and a heart rate from a user and sending advice to the user based on the obtained health-related information, thereby supporting healthcare of the user, and a healthcare apparatus and a server used for the healthcare system.

### Background Art

In recent years, it is recommended that light exercise such as walking should be done so as to maintain health. Those who walk a predetermined steps or more per day while wearing a small pedometer on a belt are increasing in number. Under this circumstance, various pedometers have been proposed and on sale.

JP2000-209142 A discloses a mobile phone with a pedometer built therein, capable of storing measured number of steps in an internal memory. This mobile phone also has a function of previously recording a user' step, calculating a walk distance from the number of steps, and displaying the calculated results on a graph. Mobile phones are likely to be carried about at all times. Therefore, such a mobile phone has an advantage that a user is not required to carry a pedometer separately.

The above-mentioned mobile phone allows a user to find the number of steps and a walk distance; however, the user cannot find if he/she is exercising appropriately. Furthermore, the user cannot take advice such as an exercise load, so that the user is required to acquire the knowledge of an exercise load and to control the state of an exercise load during exercise.

Therefore, some users cannot appropriately arrange exercise in accordance with the health condition and the state of an exercise load. Consequently, they may impose an excessive exercise load on them. Alternatively, due to an insufficient exercise load, the effects of the exercise cannot be obtained irrespective of whether a sufficient time is taken for the exercise.

Furthermore, the above-mentioned mobile phone is integrated with a pedometer so as to have the functions as the mobile phone and the pedometer. However, such a mobile phone measures the number of steps merely by using the remaining ability thereof, and does not have any effect of integration of the mobile phone with the pedometer.

The object of the present invention is to solve the above-mentioned problem and to provide: a healthcare system for providing an exercise menu so that a user can do exercise under an appropriate exercise load; a healthcare apparatus and a server used for the healthcare system; and a healthcare method.

### Disclosure of Invention

In order to achieve the above-mentioned object, the healthcare system of the present invention includes: a healthcare apparatus for obtaining health-related information of a user; and a server, wherein the healthcare apparatus transmits the obtained health-related information to the server, the server creates exercise menu information to be a target for the user to do exercise and advice information for healthcare of the user, and transmits the created exercise menu information and advice information to the healthcare apparatus, and the advice information is created based on the exercise menu information and the received health-related information.

Thus, according to the healthcare system of the present invention, the user is not required to control an exercise load by himself/herself, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily.

In the above-mentioned healthcare system of the present invention, the healthcare apparatus transmits the health-related information at an every previously set time or at an every predetermined time. Furthermore, if the healthcare apparatus obtains the health-related information during exercise of the user, the healthcare apparatus can transmit the health-related information every time obtaining the health-related information.

Furthermore, in the above-mentioned healthcare system of the present invention, the healthcare apparatus receives an input of user-related information, and transmits the user-related information to the server. In this case, the exercise menu information can be created based on the user-related information. Furthermore, the server also can create advice information based on the user-related information and the health-related information.

In order to achieve the above-mentioned object, the healthcare apparatus of the present invention obtains health-related information of a user and manages the health of the user, the healthcare apparatus including: a receiving part for receiving information from an external apparatus; a health-related information obtaining part for obtaining the health-related information of the user; a transmitting part for transmitting the obtained health-related information to the external apparatus; and a display part, wherein the receiving part receives exercise menu information to be a target for the user to do exercise, and advice information for healthcare of the user created by the external apparatus based on the exercise menu information and the transmitted health-related information, and the display part displays at least the exercise menu information and the advice information.

Thus, according to the healthcare apparatus of the present invention, the user is not required to control an exercise load by himself/herself, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily.

In the above-mentioned healthcare apparatus of the present invention, the transmitting part can transmit the health-related information at an every previously set time or at an every predetermined time. Furthermore, if the health-related information obtaining part obtains the health-related information during exercise of the user, the transmitting part transmits the health-related information every time obtaining the health-related information.

Furthermore, the above-mentioned healthcare apparatus of the present invention can have an input part for inputting user-related information, wherein the transmitting part can transmit the user-related information to the external apparatus. In this case, the exercise menu information can be created based on the user-related information. Furthermore, the advice information may be created based on the user-related information and the health-related information.

In order to achieve the above-mentioned object, the server of the present invention receives health-related information of a user to create advice information, the server including: a receiving part for receiving the health-related information of the user obtained at an external terminal apparatus; an exercise menu information creating part for creating exercise menu information to be a target for the user to do exercise; an advice information creating part for creating advice information for healthcare of the user; and a transmitting part for transmitting the exercise menu information and the advice information to the terminal apparatus, wherein the advice information creating part creates the advice information based on the received health-related information and exercise menu information.

Thus, according to the server of the present invention, the user is not required to control an exercise load by himself/herself, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily.

In the above-mentioned server of the present invention, the exercise menu information creating part can create the exercise menu information based on user-related information transmitted from the external terminal apparatus. In this case, the advice information creating part also can create advice information based on the user-related information and the health-related information.

In order to achieve the above-mentioned object, the healthcare method of the present invention uses a server and a healthcare apparatus connected to the server, the method including the steps of: (a) transmitting exercise menu information to be a target for a user to do exercise from the server to the healthcare apparatus; (b) obtaining health-related information of a user by the healthcare apparatus; (c) transmitting the obtained health-related information from the healthcare apparatus to the server; (d) creating advice information based on the health-related information and the exercise menu information by the server and transmitting the advice information to he healthcare apparatus; and (e) providing a user with the received advice information by the healthcare apparatus.

Thus, according to the healthcare method of the present invention, the user is not required to control an exercise load by himself/herself, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily.

In the above-mentioned healthcare method of the present invention, transmission of the health-related information by the healthcare apparatus can be conducted at an every previously set time or at an every predetermined time. Furthermore, if the healthcare apparatus obtains the health-related information during exercise of the user, the healthcare apparatus can transmit the health-related information every time obtaining the health-related information.

Furthermore, the above-mentioned healthcare method of the present invention may include the step of receiving an input of user-related information by the healthcare apparatus prior to the above-mentioned step (a) and transmitting the user-related information to the server. In this case, in the step (a), the server can create the exercise menu information based on the user-related information. Furthermore, in the above-mentioned step (d), the server can create advice information based on the user-related information and the health-related information.

An example of the health-related information in the present invention includes information containing at least one of a heart rate of a user and the number of steps thereof. Furthermore, an example of the exercise menu information includes information containing at least one of a target heart rate, a target number of steps, a target walking distance, a target duration, and an exercise program imposing target values. An example of the user-related information in the present invention includes information containing at least one of a purpose of exercise of a user and personal information thereof.

### Brief Description of Drawings

Figure 1 shows schematic configurations of a healthcare system, a healthcare apparatus, and a server of Embodiment 1 according to the present invention.
Figure 2 shows an example of exercise menu information transmitted from the server to the healthcare apparatus of Embodiment 1 according to the present invention.
Figure 3 is a flow chart illustrating a healthcare method of Embodiment 1 and an operation of the health care system of Embodiment 1.
Figure 4 shows schematic configurations of a healthcare system, a healthcare apparatus, and a server of Embodiment 2 according to the present invention.
Figure 5 shows schematic configurations of a healthcare system, a healthcare apparatus, and a server of Embodiment 3 according to the present invention.
Figure 6 is a flow chart illustrating a healthcare method of Embodiment 3 and an operation of the healthcare system of Embodiment 3.
Figure 7 shows the healthcare apparatus of Embodiment 3 during input of user-related information.
Figure 8 shows a healthcare apparatus of Embodiment 3 in which exercise menu information is displayed.
Figure 9 shows a schematic configuration of a healthcare apparatus of Embodiment 4 according to the present invention.
Figure 10 shows an input screen of user-related information in Embodiment 4.
Figure 11 shows a display screen of a course diagram of a walk rally in Embodiment 4.
Figure 12 shows a display screen of a golf course layout and advice information in Embodiment 4.

### Best Mode for Carrying Out the Invention

### (Embodiment 1)

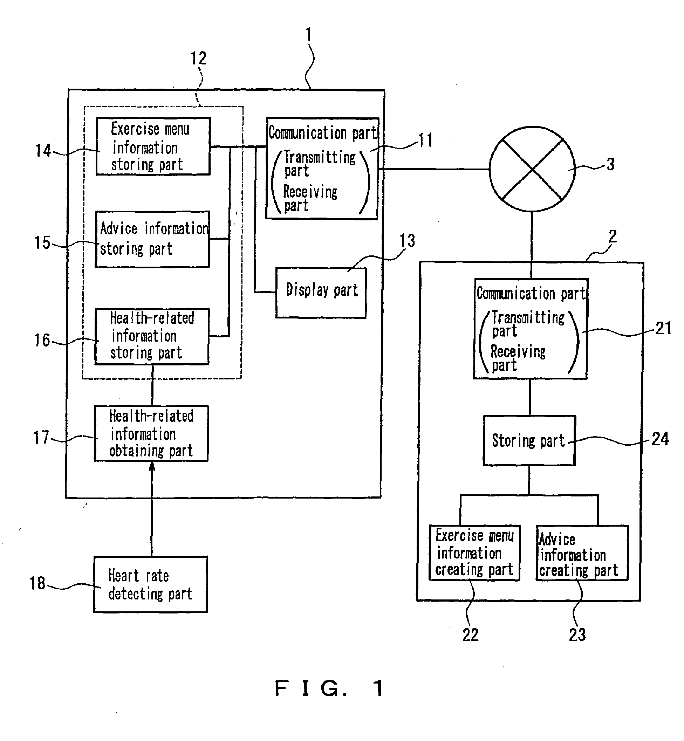

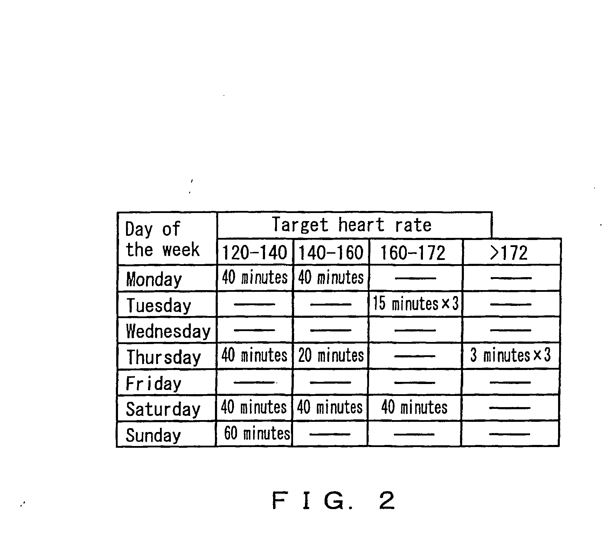

Hereinafter, a healthcare system, a healthcare apparatus, a server, and a healthcare method of Embodiment 1 according to the present invention will be described with reference to Figures 1 to 3. Figure 1 shows schematic configurations of a healthcare system, a healthcare apparatus, and a server of Embodiment 1 according to the present invention. Figure 2 shows an example of exercise menu information transmitted from the server to the healthcare apparatus in Embodiment 1.

First, the configurations of the healthcare system, the healthcare apparatus, and the server of Embodiment 1 will be described. The healthcare system of Embodiment 1 obtains a heart rate of a user during exercise as health-related information, and conducts healthcare of the user based on the obtained heart rate.

As shown in Figure 1, the healthcare system of Embodiment 1 includes a healthcare apparatus 1 of Embodiment 1 and a server 2 of Embodiment 1. In Embodiment 1, the healthcare apparatus 1 and the server 2 are connected to each other through an Internet 3.

According to the present invention, the connection between the healthcare apparatus 1 and the server 2 is not necessarily through the Internet 3. Any communication net capable of transmitting/receiving information may be used. The healthcare apparatus 1 and the server 2 may be connected to each other directly, for example, through a telephone line. Alternatively, they may be connected to each other through a cable or radio other than the telephone line.

The healthcare apparatus 1 of Embodiment 1 includes a communication part 11, a storing part 12, a display part 13, a health-related information obtaining part 17, and a heart rate detecting part 18. Furthermore, the healthcare apparatus 1 of Embodiment 1 is designed so as to be carried by a user. In the case where a space for exercise is limited as in a sports gym, the healthcare apparatus 1 may be of a standstill type.

The communication part 11 functions as a receiving part for receiving information transmitted from the server 2 through the Internet 3, and as a transmitting part for transmitting information to the server 2 through the Internet 3.

The storing part 12 stores information received in the communication part 11 and information obtained in the healthcare-related information obtaining part 17. The storing part 12 includes an exercise menu information storing part 14, an advice information storing part 15, and a health-related information storing part 16. The contents of each information will be described later.

The display part 13 displays information transmitted from the server, such as exercise menu information and advice information, obtained health-related information of a user, and the other information required by the user

The health-related information obtaining part 17 obtains health-related information of a user. In the present embodiment, since a heart rate is used as health-related information as described above, the health-related information is obtained by using a heart rate detecting part 18. The heart rate detecting part 18 is formed separately from the health-care apparatus 1, and is attached to a user's body via a belt or the like.

Upon detecting a user's heart rate, the heart rate detecting part 18 transmits the detected heart rate to the health-related information obtaining part 17 through a weak electromagnetic wave. Thereafter, the detected heart rate is converted to heart rate data by the health-related information obtaining part 17, and heart rate data is stored in the health-related information storing part 16 as health-related information. In the present embodiment, although conversion to heart rate data is conducted by measuring an interval between the detected heart rates, the present invention is not limited thereto.

The heart rate data stored in the health-related information storing part 16 is transmitted to the server 2 by the communication part 11. In Embodiment 1, the heart rate data is transmitted to the server 2 at a time (e.g., 3 p.m.) previously set by a user. In the health-related information storing part 16, heart rate data until the set time is accumulated.

Transmission of health-related information in the present invention is not limited to the above example, and can be set appropriately in accordance with the kind of health-related information and the exercise conducted by a user. In the present invention, health-related information may be transmitted at a predetermined time interval (e.g., every one hour, every two hours). Furthermore, in the case where health-related information is obtained during exercise of a user, the health-related information may be transmitted every time it is obtained (i.e., in real time).

The server 2 of Embodiment 1 includes a communication part 21, a storing part 24, an exercise menu information creating part 22, and an advice information creating part 23. The communication part 21 functions as a receiving part and a transmitting part in the same way as in the communication part 11. The health-related information (heart rate data) transmitted from the healthcare apparatus 1 is received by the communication part 21 and stored in the storing part 24.

The exercise menu information creating part 22 creates exercise menu information to be a target for a user to do exercise. The created exercise menu information is stored in the storing part 24, and thereafter, transmitted to the healthcare apparatus 1 by the communication part 21. The user will do exercise based on the exercise menu information.

The exercise menu information in Embodiment 1 contains a target heart rate and a target duration for allowing the target heat rate to be continued, as shown in Figure 2. The present invention is not limited thereto. The exercise menu information may contain, for example, an exercise program imposing target values such as 50 strokes of tennis balls against a wall, 20 km of cycling, and 1 km of swimming. Furthermore, the exercise menu information may contain a target number of steps and a target walk distance. In this case, the exercise menu information further may contain a recommended route.

The advice information creating part 23 creates advice information based on the health-related information received by the communication part 21 and the exercise menu information created by the exercise menu information creating part 22. The created advice information is stored in the storing part 24, and thereafter, transmitted to the healthcare apparatus 1 by the communication part 21. The user can conduct his/her healthcare based on this advice information.

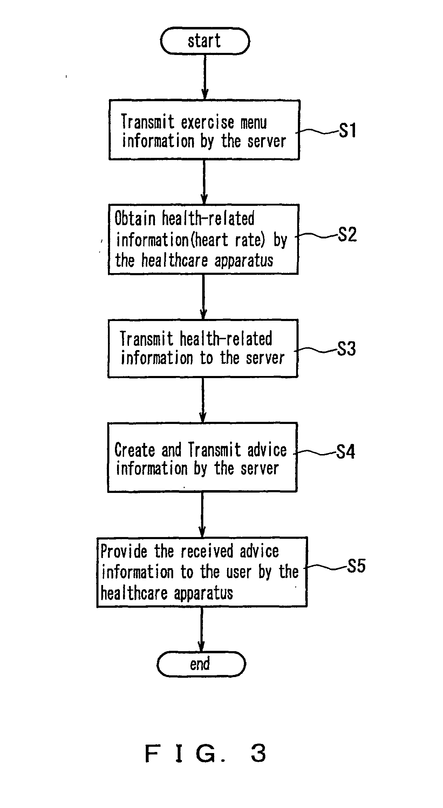

Next, the healthcare method of Embodiment 1 and an operation of the healthcare system of Embodiment 1 will be described. Figure 3 is a flow chart illustrating the healthcare method of Embodiment 1 and an operation of the healthcare system of Embodiment 1.

As shown in Figure 3, first, exercise menu information is transmitted to the healthcare apparatus 1 by the server 2 (Step S1) The transmitted exercise menu information is once stored in the exercise menu information storing part 14, and displayed on the display part 13. Transmission from the server 2 is conducted at a time previously set by the user.

For example, in the case of Monday, a training menu on Monday shown in Figure 2 is transmitted as exercise menu information, and stored in the exercise menu information storing part 14 (Step S1). The display part 13 displays "40 minutes of exercise in a heart rate of 120 to 140 beats per min., and 40 minutes of exercise in a heart rate of 140 to 160 beats per min". The user does exercise using the displayed menu as a target.

Next, when the user attaches the heart rate detecting part 18 to his/her body, and does exercise based on the displayed exercise menu, the health-related information obtaining part 17 obtains heart rate data that is health-related information (Step S2). The obtained heart rate data is once stored in the health-related information storing part 16.

Thereafter, at a previously set time (e.g., 3 p.m.), the communication part 11 transmits the heart rate data stored in the health-related information storing part 16 to the server 2 (Step S3). The transmitted heart rate data is stored in the storing part 24 of the server 2.

Next, the server 2 creates advice information and transmits it to the healthcare apparatus 1 (Step S4). More specifically, the advice information is created by the advice information creating part 23, based on the heart rate data and the exercise menu information stored in the storing part 24.

For example, in the case where the transmitted heart rate data is "45 minutes of exercise in a heart rate of 120 to 140 beats per min., and 20 minutes of exercise in a heart rate 140 to 160 beats per min.", the advice information creating part 23 compares the heart rate data with the training menu on Monday to determine that at least 20 minutes of exercise in a heart rate of 140 to 160 beats per min. is required.

Then, the advice information creating part 23 creates advice information: "at least 20 minutes of exercise in a heart rate of 140 to 160 beats per min. is required". Thereafter, the advice information is transmitted from the communication part 21 and received by the communication part 11 of the healthcare apparatus 1, and the advice information is once stored in the advice information storing part 15.

Next, the display part 13 of the healthcare apparatus 1 displays that "Please conduct at least 20 minutes of training under an exercise load in a heart rate of 140 to 160 beats per min.", thereby providing the advice information to the user (Step S5). The user conducts further training based the advice information, thereby conducting his/her healthcare easily.

On the subsequent Tuesday, a training menu on Tuesday shown in Figure 2 is transmitted from the server 2 as an exercise menu information, and stored in the exercise menu information storing part 14 (Step S1). The display part 13 displays that "15 minutes of exercise in a heart rate of 160 to 172 beats per min. should be done three times".

The user attaches the heart rate detecting part 18 to his/her body in the same way as on Monday, and does exercise based on the displayed exercise menu. Thereafter, health-related information is obtained and transmitted (Steps S2 and S3), and advice information is created and transmitted (Step S4), whereby the advice information is provided to the user (Step S5).

If the contents of the health-related information obtained from the user are, for example, "15 minutes of exercise in a heart rate of 160 to 172 beats per min. should be done twice, and 25 minutes of exercise in a hart rate of at least 173 beats per min. should be done once", the display part 13 displays, as advice information, a message describing that "A load larger than the plan is imposed today. Take a sufficient rest".

Furthermore, in this case, the server 2 transmits recommended warm-down stored in the storing part 24 to the health-care apparatus 1 as an animation file. The healthcare apparatus 1 reproduces the received animation file, and displays an image of the recommended warm-down on the display part 13. Accordingly, the user can do appropriate warm-down by himself/herself.

On the subsequent Wednesday to Sunday, Steps S1 to S5 described above are performed, and the user can receive advice from the server 2. Therefore, the user can conduct his/her healthcare.

Thus, in Embodiment 1, the user is not required to control an exercise load, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily.

### (Embodiment 2)

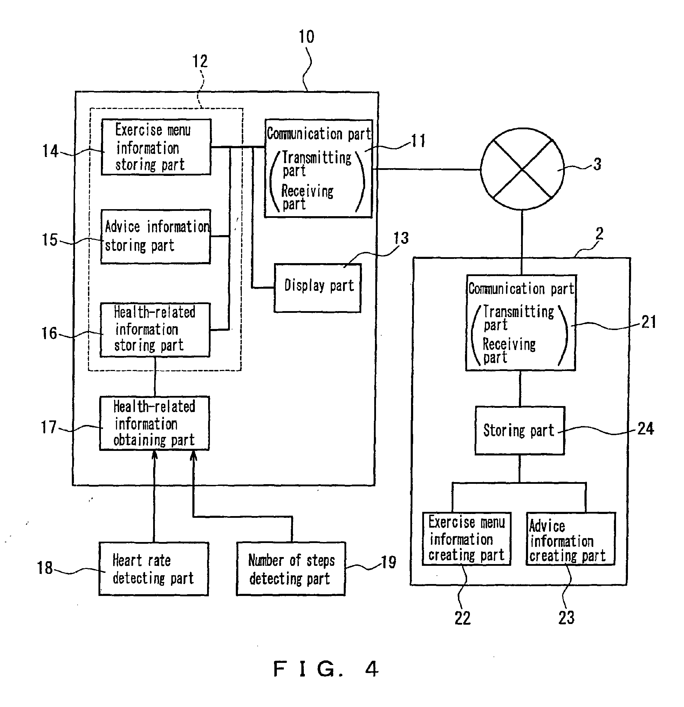

Next, a healthcare system, a healthcare apparatus, a server, and a healthcare method of Embodiment 2 according to the present invention will be described with reference to Figure 4. Figure 4 shows schematic configurations of the healthcare system, the healthcare apparatus, and the server of Embodiment 2 according to the present invention. Among the reference numerals in Figure 4, those which are the same as those in Figure 1 denote the same components as those in Figure 1.

As shown in Figure 4, the healthcare system of Embodiment 2 includes a healthcare apparatus 10 and a server 2. The server 2 of Embodiment 2 is the same as that of Embodiment 1. However, unlike Embodiment 1, the healthcare apparatus 10 of Embodiment 2 has a number of steps detecting part 19. The remaining parts are configured in the same way as in the healthcare apparatus of Embodiment 1. The healthcare apparatus 10 of Embodiment 2 also is configured so as to be carried by a user.

Furthermore, the healthcare method of Embodiment 2 is the same as that of Embodiment 1, except that the health-related information obtaining part 17 obtains heart rate data and the number of steps data as healthcare information in Step S2 shown in Figure 3, and transmit it to the server 2. According to the healthcare method of Embodiment 2, processing also is conducted in accordance with Steps S 1 to S5 shown in Figure 3.

The number of steps detecting part 19 also can be configured separately from the healthcare apparatus 10 in the same way as in the heart rate detecting part 18. In this case, the number of steps detecting part 19 also is attached to a user's body through a belt or the like, and a detected vibration during walking is transmitted to the health-related information obtaining part 17 through a weak electromagnetic wave. Furthermore, the number of steps detecting part 19 also can be built in the healthcare apparatus 10.

The vibration during walking detected by the number of steps detecting part 19 is transmitted to the health-related information obtaining part 17, and converted to the number of steps data by the health-related information obtaining part 17. The number of steps data is stored in the health-related information storing part 16 in the same way as in heart rate data, and transmitted to the server 2 by the communication part 11 every predetermined set time.

In Embodiment 2, in order for the server 2 to calculate a walking pace of a user, a time between the vibrations during walking also is transmitted to the server 2 as data.

In Embodiment 2, heart rate data and number of steps data are obtained as health-related information. Therefore, the exercise menu created by the server 2 is different from that of Embodiment 1. The exercise menu information in Embodiment 2 contains a target heart rate, a target number of steps, and a target time for achieving them, such as "walking at least 10000 steps in a heart rate of 90 to 110 beats per min. per day".

Furthermore, in Embodiment 2, health-related information is transmitted to the server 2 even prior to the passage of a target time (one day in the above example), and advice information is created with respect to the health-related information at this time. For example, heart rate data and number of steps data stored in the health-related information storing part 16 are transmitted, for example, in the middle (e.g., 3 p.m.) of one day.

At this time, in the case where the number of steps data is 4000 steps with which the target of one day may not be achieved, advice informations "Today's number of steps is small. Try to walk a little bit faster if the target number of steps may not be achieved" is created by the advice information creating part 23 and transmitted to the healthcare apparatus 10.

Thus, in Embodiment 2, the user is not required to control an exercise load by himself/herself, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily.

Furthermore, in Embodiment 2, the user can receive advice information during exercise as well as after exercise. Therefore, the user can do exercise while confirming the achieved degree of the target.

### (Embodiment 3)

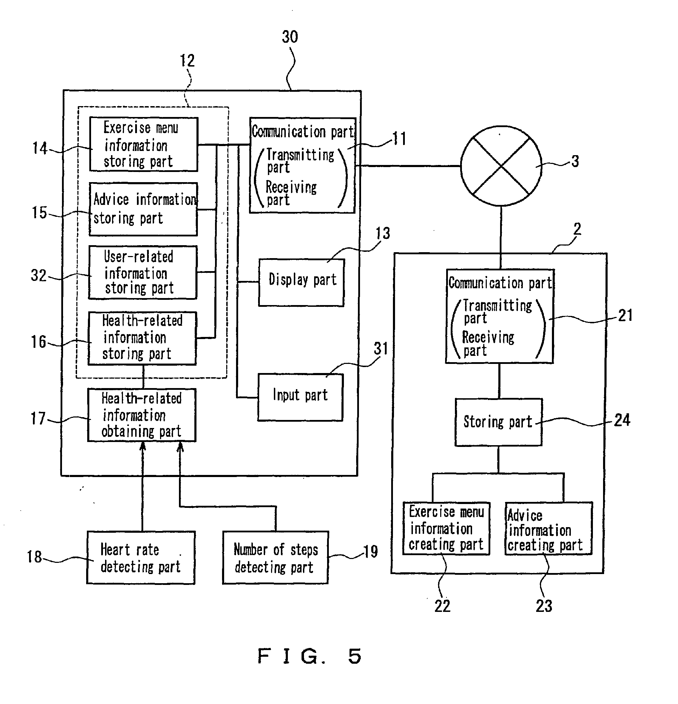

Next, a healthcare system, a healthcare apparatus, a server, and a healthcare method of Embodiment 3 according to the present invention will be described with reference to Figures 5 to 8. Figure 5 shows schematic configurations of the healthcare system, the health care apparatus and the server of Embodiment 3 according to the present invention. Among reference numerals in Figure 5, those which are the same as those in Figure 1 denote the same components as those in Figure 1.

First, the healthcare system, the healthcare apparatus, and the server of Embodiment 3 will be described.

As shown in Figure 5, the healthcare system of Embodiment 3 includes a healthcare apparatus 30 and a server 2. Unlike Embodiments 1 and 2, the healthcare apparatus 30 of Embodiment 3 has an input part 31 for inputting user-related information and a user-related information storing part 32. The remaining parts are configured in the same way as in the healthcare apparatus of Embodiment 2. The healthcare apparatus 30 of Embodiment 3 also is configured so as to be carried by a user.

Examples of the user-related information in the present invention include: a purpose of exercise of a user such as keeping of health, strengthening of muscular power, and diet; user's personal information such as a height, a weight, an age, a body fat percentage, a step, and upper and lower limit values of a heart rate; and the like.

The user-related information input in the input part 31 is stored in the user-related information storing part 32, and thereafter, transmitted to the server 2 by the communication part 11. The user-related information received by the communication part 21 of the server 2 is stored in the storing part 24 of the server 2.

Furthermore, the server 2 of Embodiment 3 is configured in the same way as in the server 2 of Embodiment 1; however, it is different from the server 2 of Embodiment 1 in that user-related information is utilized in creating exercise menu information. In Embodiment 3, the exercise menu information creating part 22 creates an exercise menu based on the user-related information stored in the storing part 24.

For example, in the case where the user-related information represents that a purpose of exercise of a user is to keep health, the exercise menu information creating part 22 creates exercise menu information to such a degree that user' physical strength is not decreased, in accordance with a body structure and an age. Furthermore, in the case where a purpose of exercise of a user is diet, the exercise menu information creating part 22 creates exercise menu information for a calorie consumption larger than that of the case of keeping health.

Therefore, in the present embodiment, the user can do exercise in accordance with his/her purpose of exercise, which enables the user to obtain effects without fail and to avoid giving up the exercise halfway therethrough and stopping it.

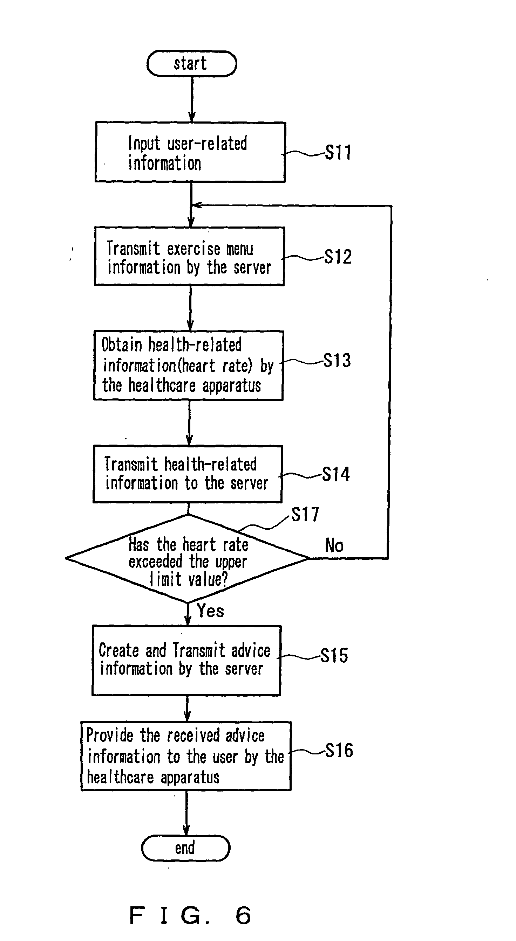

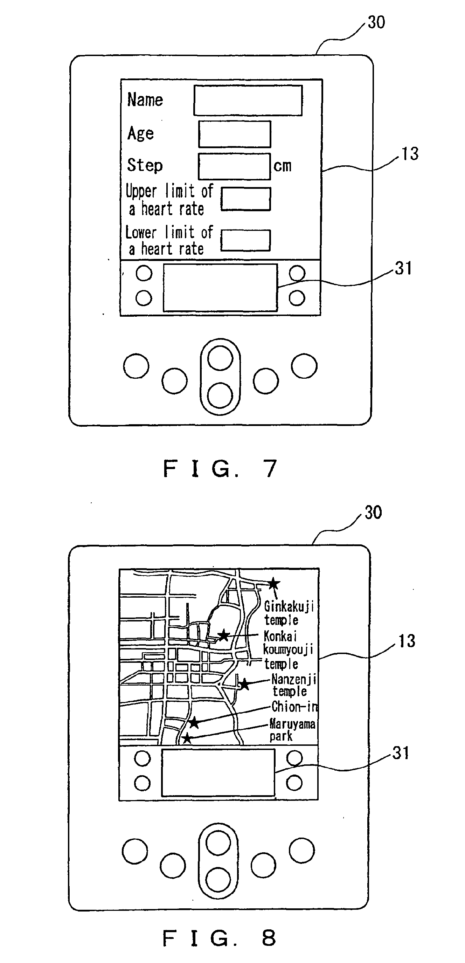

Next, the healthcare method of Embodiment 3 and an operation of the healthcare system of Embodiment 3 will be described. Figure 6 is a flow chart illustrating the healthcare method of Embodiment 3 and the operation of the healthcare system of Embodiment 3. Figure 7 shows the healthcare apparatus of Embodiment 3 during input of user-related information. Figure 8 shows the healthcare apparatus of Embodiment 3 in which exercise menu information is displayed.

As shown in Figure 6, first, the healthcare apparatus 30 receives an input of user-related information from a user (Step S11). More specifically, as shown in Figure 7, an input screen is displayed on the display part 13 of the healthcare apparatus 30. The user inputs a name, an age, a step, and upper and lower limit values of a target heart rate during exercise through a dedicated pen. Although not shown in Figure 7, a purpose of exercise and a user's address also are input on the subsequent screen.

The healthcare apparatus of Embodiment 3 has a function of automatically setting upper and lower limit values of a heart rate during exercise, based on an age. Therefore, the user is not required to input upper and lower limit values of a target heart rate directly. However, in the case where there is an instruction of a medical practitioner such as a doctor, regarding upper and lower limit values of a target heart rate, the user inputs the instructed value.

Furthermore, in Embodiment 3, as an input method to the input part 31, a pen input is used. However, the present invention is not limited thereto. According to the present invention, inputs other than a pen input, such as a key input and a voice input, also may be used,

Next, the input user-related information is transmitted to the server 2, and stored in the storing part 24 of the server 2. Then, exercise menu information is created by the exercise menu information creating part 22 based on the user-related information, and transmitted to the healthcare apparatus 30 (Step S12).

In Embodiment 3, the exercise menu information is "4 km of walking". The exercise menu information includes a recommended route in accordance with a user's address. As shown in Figure 8, a recommended route showing a starting point, a check point, and a destination is displayed on the display part 13 of the healthcare apparatus 30. In Embodiment 3, the case where a user's address lies in Kyoto is assumed. As shown in Figure 8, a recommended route from Maruyama park (starting point) to Ginkakuji temple via Chion-in, Nanzennji temple, and Konkai koumyouji temple is displayed

Next, when the user attaches the heart rate detecting part 18 and the number of steps detecting part 19 to his/her body, and starts walking along the displayed recommended route, the health-related information obtaining part 17 obtains health-related information (heart rate data and number of steps data) (Step S13).

In Embodiment 3, the obtained heart rate data and number of steps data are stored in the health-related information storing part 16 and simultaneously transmitted to the server 2 by the communication part 11, whereby they are stored in the storing part 24 of the server 2 (Step S14). Furthermore, time data (walking pace) between vibrations during walking also is stored in the storing part 24 of the server 2.

Next, the advice information creating part 23 compares the stored heart rate data with the upper limit value of a heart rate contained in user-related information to determine if the user's heart rate exceeds the upper limit value (Step S17). In the where the user's heart rate exceeds the upper limit value, the advice information creating part 23 creates advice information that the heart rate exceeds the upper limit value, such as "Take a rest for about 10 minutes" or "Walk more slowly", and the advice information is transmitted to the healthcare apparatus 30 (Step S15).

Thereafter, the display part 13 of the healthcare apparatus 30 displays that "Take a rest for about 10 minutes" or "Walk more slowly", whereby the advice information is provided to the user (Step S16). The healthcare apparatus 30 can be configured so as to utter a warning sound, in the case of displaying the advice information to be warning to the user.

In the case where the user reaches the destination, the advice information creating part 23 calculates a distance which the user has walked, from the number of steps data and user's step stored in the storing part 24, and roughly calculates a calorie consumption of the user from the heart rate data, the number of steps data, and the time data (walking pace) between vibrations during walking stored in the storing part 24. The calculated distance and calorie consumption are transmitted to the healthcare apparatus 30 as advice information.

Furthermore, the advice information creating part 23 creates advice information that "Try to clear the target tomorrow" or "Increase an exercise load a little bit more tomorrow". These pieces of information also are transmitted to the healthcare apparatus 30.

Thereafter, the display part 13 of the healthcare apparatus 30 displays a walking distance and a calorie consumption, together with a message that "Try to clear the target tomorrow" or "Increase an exercise load a little bit more tomorrow".

Thus, in Embodiment 3, the user is not required to control an exercise load by himself/herself, and the exercise load on the user is managed at all times. Therefore, the user easily can avoid the problems that too much exercise load is imposed, effects cannot be obtained due to an insufficient exercise load, etc., as in the prior art, whereby the user can conduct healthcare easily. Furthermore, in Embodiment 3, the health condition of the user during exercise is controlled, so that the user can do exercise with a feeling of security

### (Embodiment 4)

Next, a healthcare apparatus and a healthcare method of Embodiment 4 according to the present invention will be described.

Conventionally, when playing a sports competition, in particular, a personal competition such as jogging and golf, a player generally plays a competition while determining his/her athletic ability and the situation such as the weather.

In jogging, a player does exercise while monitoring a heart rate with a heart rate meter and controlling the degree of exercise so that the heart rate comes in a predetermined range. In golf, a caddy gives advice to a player while considering the course situation such as the positions of a bunker and the pond, and the distances to a putting green and a pin, and a wind direction.

Although a player can adjust the degree of exercise by monitoring a heart rate or the like, the player cannot obtain information regarding how to address the situation where the heart rate exceeds the upper limit value.

Furthermore, in golf, although a caddy gives advice to a player, the caddy may not exactly grasp individual athletic ability of the player. Thus, it cannot be considered that the player can get appropriate advice.

The healthcare apparatus of Embodiment 4 solves the above-mentioned problems, which includes: a competition information storing part for storing information on a sports competition; a player personal information storing part for storing information on a player; and an advice information generating part for generating advice information on a competition to a player, based on the information on a sports competition stored in the competition information storing part and the player personal information of the player stored in the player personal information storing part.

More preferably, the player personal information is at least one of skill information on an athletic skill of a player and health information on the health of the player.

Furthermore, the healthcare method of Embodiment 4 includes storing information on a sports competition in a competition information storing part; storing information on a player in a player personal information storing part; and generating advice information on a competition to a player, based on the information on a sports competition stored in the competition information storing part and the player personal information of the player stored in the player personal information storing part.

More preferably, the personal information stored in the personal information storing part is at least one of skill information on an athletic skill of a player and health information on the health of the player.

According to the healthcare apparatus and the healthcare method of Embodiment 4, information (competition personal information) on athletic ability of an individual player is stored in an apparatus, and a player can be given advice based on the player personal information. Therefore, advice appropriate for a player can be given to the player, and the player can play a competition in an optimum state.

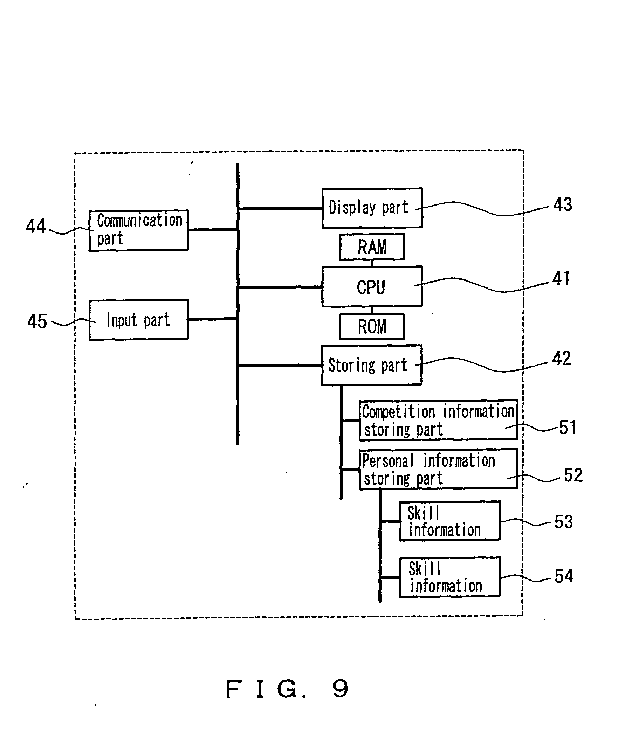

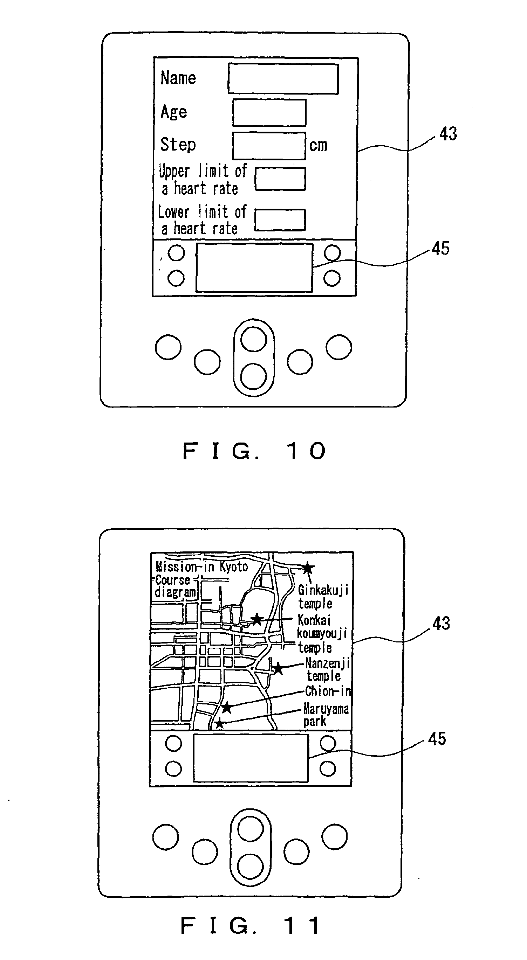

Next, an example using the healthcare apparatus of Embodiment 4 for a walk rally will be described. Figure 9 shows a schematic configuration of the healthcare apparatus of Embodiment 4 according to the present invention. Figure 10 shows an input screen of user-related information in Embodiment 4. Figure 11 shows a display screen of a course diagram of the walk rally in Embodiment 4.

A participant (user of the healthcare apparatus) participates in a walk rally, carrying a portable terminal apparatus (healthcare apparatus) including a display part 43, an input part 45, a CPU 41, a storing part 42, and a communication part 44 for communication with an outside as shown in Figure 9. The storing part 42 of the portable terminal apparatus includes a competition information storing part 51 and a personal information storing part 52, and the personal information storing part 52 is composed of a skill information storing part 53 and a health information storing part 54.

As shown in Figure 10, the participant previously inputs a name, an age, a step, and the upper and lower limit values of a target heart rate during exercise in the portable terminal apparatus with a pen input unit through the input part 45. The upper and lower limit values of a heart rate during exercise are determined by automatic setting and manual setting. According to the automatic setting, the upper and lower limit values obtained from an age are set automatically. According to the manual setting, the upper and lower limit values are set arbitrarily in accordance with an instruction of a medical practitioner such as a doctor.

Upon reaching a destination (Maruyama park) of the walk rally, the participant downloads course information of the walk rally shown in Figure 11 from a data transmission terminal apparatus (not shown) disposed at a starting point of the walk rally to the portable terminal apparatus, thereby storing the course information in the competition information storing part 51 of the storing part 42. The course information contains a walk rally course (course from Maruyama park (starting point) to Ginkakuji temple via Chion-in, Konkai koumyouji temple, Nanzenji temple, with each temple being a check point) and a program for recording answers to quizzes given at check points and transmitting them to a server (not shown) that is a walk rally recording management apparatus.

In the present example, the walk rally course is downloaded from the data transmission terminal apparatus disposed at the starting point. However, the walk rally course also can be downloaded in real time during exercise through the Internet or a mobile phone.

The participant carries a heart rate meter and a pedometer capable of transmitting heart rate data and number of steps data to the portable terminal apparatus through a weak electromagnetic field. The heart rate meter and the pedometer transmit heart rate data and number of steps data during the walk rally to the communication part 44 of the portable terminal apparatus, and these data are recorded in the health information storing part 54 of the storing part 42 of the portable terminal apparatus.

In the case where a heart rate during the walk rally exceeds the upper limit value of the previously set heart rate, the portable terminal apparatus utters a warning sound to notice the participant that the heart rate exceeds the upper limit value, and displays advice on the display part 43 of the portable terminal apparatus in accordance with the participant's state: "Take a rest for about 10 minutes" or "Walk more slowly".

The participant records an answer to a quiz given at each check point in the portable terminal apparatus while walking around Chion-in, Konkai koumyouji temple, and Nanzenji temple, and proceeds to the subsequent check point. At Ginkakuji temple that is the last check point, the participant transmits the answers to the quizzes given at the respective check points, heart rate data, and number of steps data to the walk rally recording management apparatus (server).

From the results obtained by summarizing the answers to the quizzes, the walk rally recording management apparatus specifies a record winner to be given a gift, and randomly selects a person to be given a gift by a lottery. Furthermore, the heart rate data and the number of steps data are recorded in a participant personal data recording region of the walk rally recording management apparatus, and also can be used as data of a report of kinetotherapy to a family doctor.

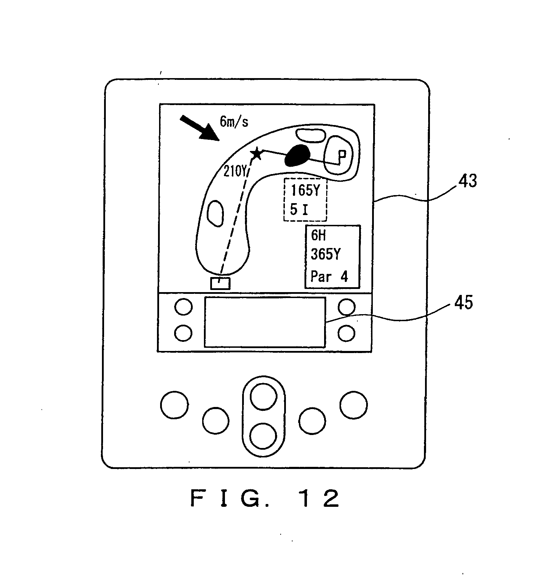

Next, an example using the healthcare apparatus of Embodiment 4 for golf will be described. Figure 12 is a display screen showing a golf course layout and advice information in Embodiment 4.

A golf player (user) downloads course information such as a course layout, and the positions of a pin and a tee from a course data providing apparatus (not shown) to the portable terminal apparatus at a clubhouse of a golf course, and stores the course information in the competition information storing part 51 of the storing part 42.

Furthermore, at a tee position on each hole, there is an information sending apparatus (not shown). The communication part 44 of the portable terminal apparatus receives weather information such as a wind direction, a wind force, a temperature, a humidity, and the like from the information sending apparatus through a weak electromagnetic wave.

The golf player inputs personal information on the golf player such as a flying distance at each count, a player's peculiar way of stroking a ball, and the like, and stores it in the skill information storing part 53 of the storing part 42 of the portable terminal apparatus. The golf player may input a flying distance from his/her experience. Alternatively, the golf player may test-stroke balls at a driving range before starting; measure and analyze a relationship between a count and a flying distance, and a player's peculiar way of stroking a ball with image measuring means or the like; and input the obtained result in the portable terminal apparatus.

The golf player allows the screen of the portable terminal apparatus to display a course diagram at a tee position. The golf player captures the current weather information into the portable terminal apparatus from the information sending apparatus disposed at the tee position. The portable terminal apparatus analyzes the weather information such as a wind direction, a wind force, a temperature and a humidity, and a course layout and personal information on a golf player previously captured in the portable terminal apparatus and displays, as advice information, a count of a club and a direction for which a tee shot is targeted on the screen of the portable terminal apparatus.

The golf player selects a club with reference to the advice information displayed on the screen and strokes a tee shot. The golf player moves to a second stroke position, and indicates the position (" 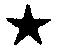 " in Figure 12) of a ball with a pen on the course layout displayed in the potable terminal apparatus, as shown in Figure 12. On the course layout of the display part 43 of the portable terminal apparatus, the position of a ball is displayed, and a flying distance of a tee shot, the remaining distance to a pin, weather information such as a wind direction, the remaining distance, and the count of a recommended club.

Thereafter, the golf player continues to play golf while recording the position of a ball at each stroke until 18 holes. The number of putts (strokes) is input through the input part 45 of the portable terminal apparatus after the completion of the hole.

The portable terminal apparatus summarizes the total number of strokes after the completion of a competition; calculates and records not only scores but also a flying distance of a tee shot, a fareway keeping ratio, an average putter number, an OB number, a bunker stroke number, a birdie number, an eagle number, a bogey number, and the like; and displays them on the display part 43 of the portable terminal apparatus. The golf player can refer to these pieces of information during the subsequent competition.

In the present example, although the course information is downloaded at a clubhouse on the date of a competition, it may be obtained through the Internet by the previous day. Furthermore, by disclosing the results of a competition, a multi-competition can be performed.

The healthcare system, the healthcare apparatus, the server, and the healthcare method according to the present invention have a main object of supporting healthcare of a user. Furthermore, they have an object of enhancing athletic ability of a user, and allowing a user to enjoy exercise.

### Industrial Applicability

As described above, the healthcare system, the healthcare apparatus, the server, and the healthcare method according to the present invention not only allow data to be obtained from a user, but also give appropriate exercise menu and advice to a user. Therefore, the user can avoid the problems that too much exercise load is imposed, effects cannot be obtained due to an insufficient exercise load, etc. Thus, the user can do appropriate exercise while keeping health. Furthermore, compared with the prior art, effects are likely to be obtained, so that the user is unlikely to give up the exercise halfway therethrough, compared with the prior art.

## Claims

1. A healthcare system comprising: a healthcare apparatus for obtaining health-related information of a user; and a server,
wherein the healthcare apparatus transmits the obtained health-related information to the server,
the server creates exercise menu information to be a target for the user to do exercise and advice information for healthcare of the user, and transmits the created exercise menu information and advice information to the healthcare apparatus, and
the advice information is created based on the exercise menu information and the received health-related information.

2. Ahealthcare system according to claim 1, wherein the healthcare apparatus transmits the health-related information at an every previously set time or at an every predetermined time.

3. A healthcare system according to claim 1, wherein the healthcare apparatus obtains the health-related information during exercise of the user, and the healthcare apparatus transmits the health-related information every time obtaining the health-related information.

4. A healthcare system according to claim 1, wherein the health-related information contains at least one selected from the group consisting of a heart rate of a user and the number of steps thereof.

5. Ahealthcare system according to claim 1, wherein the exercise menu information contains at least one selected from the group consisting of a target heart rate, a target number of steps, a target walking distance, a target duration, and an exercise program imposing target values.

6. A healthcare system according to claim 1, wherein the healthcare apparatus receives an input of user-related information, and transmits the user-related information to the server, and
the server creates the exercise menu information based on the user-related information.

7. A healthcare system according to claim 6, wherein the user-related information contains at least one selected from the group consisting of a purpose of exercise of the user and personal information thereof.

8. A healthcare system according to claim 1, wherein the server creates advice information based on the user-related information and the health-related information.

9. Ahealthcare apparatus for obtaining health-related information of a user and managing the health of the user, comprising:
a receiving part for receiving information from an external apparatus; a health-related information obtaining part for obtaining the health-related information of the user; a transmitting part for transmitting the obtained health-related information to the external apparatus; and a display part,
wherein the receiving part receives exercise menu information to be a target for the user to do exercise, and advice information for healthcare of the user created by the external apparatus based on the exercise menu information and the transmitted health-related information, and
the display part displays at least the exercise menu information and the advice information.

10. Ahealthcare apparatus according to claim 9, wherein the transmitting part transmits the health-related information at an every previously set time or at an every predetermined time.

11. A healthcare apparatus according to claim 9, wherein the health-related information obtaining part obtains the health-related information during exercise of the user, and the transmitting part transmits the health-related information every time obtaining the health-related information.

12. Ahealthcare apparatus according to claim 9, wherein the health-related information contains at least one selected from the group consisting of a heart rate of a user and the number of steps thereof.

13. A healthcare apparatus according to claim 9, wherein the exercise menu information contains at least one selected from the group consisting of a target heart rate, a target number of steps, a target walking distance, a target duration, and an exercise program imposing target values.

14. A healthcare apparatus according to claim 9, comprising an input part for inputting user-related information, wherein the transmitting part transmits the user-related information to the external apparatus, and the exercise menu information is created based on the user-related information.

15. Ahealthcare apparatus according to claim 14, wherein the user-related information contains at least one selected from the group consisting of a purpose of exercise of the user and personal information thereof.

16. Ahealthcare apparatus according to claim 14, wherein the advice information is created based on the user-related information and the health-related information.

17. A server for receiving health-related information of a user to create advice information, comprising:
a receiving part for receiving the health-related information of the user obtained at an external terminal apparatus; an exercise menu information creating part for creating exercise menu information to be a target for the user to do exercise; an advice information creating part for creating advice information for healthcare of the user; and a transmitting part for transmitting the exercise menu information and the advice information to the terminal apparatus,
wherein the advice information creating part creates the advice information based on the received health-related information and exercise menu information.

18. A server according to claim 17, wherein the health-related information contains at least one selected from the group consisting of a heart rate of a user or the number of steps thereof.

19. A server according to claim 17, wherein the exercise menu information contains at least one selected from the group consisting of a target heart rate, a target number of steps, a target walking distance, a target duration, and an exercise program imposing target values.

20. A server according to claim 17, wherein the exercise menu information creating part creates the exercise menu information based on user-related information transmitted from the external terminal apparatus.

21. A server according to claim 20, wherein the user-related information contains at least one selected from the group consisting of a purpose of exercise of the user and personal information thereof.

22. A server according to claim 20, wherein the advice information creating part creates advice information based on the user-related information and the health-related information.

23. Ahealthcare method using a server and a healthcare apparatus connected to the server, comprising the steps of
(a) transmitting exercise menu information to be a target for a user to do exercise from the server to the healthcare apparatus;
(b) obtaining health-related information of a user by the healthcare apparatus;
(c) transmitting the obtained health-related information from the healthcare apparatus to the server;
(d) creating advice information based on the health-related information and the exercise menu information by the server and transmitting the advice information to he healthcare apparatus; and
(e) providing a user with the received advice information by the healthcare apparatus.

24. A healthcare method according to claim 23, wherein transmission of the health-related information by the healthcare apparatus is conducted at an every previously set time or at an every predetermined time.

25. A healthcare method according to claim 23, wherein the healthcare apparatus obtains the health-related information during exercise of the user, and the healthcare apparatus transmits the health-related information every time obtaining the health-related information.

26. Ahealthcare method according to claim 23, wherein the health-related information contains at least one selected from the group consisting of a heart rate of a user and the number of steps thereof.

27. Ahealthcare method according to claim 22, wherein the exercise menu information contains at least one selected from the group consisting of a target heart rate, a target number of steps, a target walking distance, a target duration, and an exercise program imposing target values.

28. A healthcare method according to claim 23, wherein the healthcare apparatus receives an input of user-related information prior to the step (a), and
the server creates the exercise menu information based on the user-related information in the step (a).

29. Ahealthcare method according to claim 28, wherein the user-related information contains at least one selected from the group consisting of a purpose of exercise of the user and personal information thereof.

30. Ahealthcare method according to claim 28, wherein the server creates advice information based on the user-related information and the health-related information in the step (d).
